(19) Europäisches Patentamt
European Patent Office
Office européen des brevets

(11) EP 2 600 150 B1

# (12) EUROPEAN PATENT SPECIFICATION

(45) Date of publication and mention of the grant of the patent:
**07.01.2015 Bulletin 2015/02**

(51) Int Cl.:
*G01N 33/50* (2006.01) *C12Q 1/48* (2006.01)

(21) Application number: **12195000.0**

(22) Date of filing: **30.11.2012**

(54) **Method for determining sensitivity of tumor cells to dasatinib and computer program**

Verfahren zur Bestimmung der Empfindlichkeit von Tumorzellen auf Dasatinib und Computerprogramm

Procédé de détermination de la sensibilité de cellules tumorales au dasatinib et programme informatique

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.12.2011 JP 2011263884**

(43) Date of publication of application:
**05.06.2013 Bulletin 2013/23**

(73) Proprietor: **Sysmex Corporation**
**Kobe-shi, Hyogo 651-0073 (JP)**

(72) Inventors:
- **Kawai, Masaaki**
  **Hyogo 651-0073 (JP)**
- **Torikoshi, Yasuhiro**
  **Hyogo 651-0073 (JP)**
- **Gohda, Keigo**
  **Hyogo 651-0073 (JP)**
- **Ishihara, Hideki**
  **Hyogo 651-0073 (JP)**

(74) Representative: **Hoffmann Eitle**
**Patent- und Rechtsanwälte PartmbB**
**Arabellastraße 30**
**81925 München (DE)**

(56) References cited:
**WO-A2-2007/059430 WO-A2-2008/089135**
**WO-A2-2009/094556 WO-A2-2010/020619**

- **S. MOULDER ET AL: "Development of Candidate Genomic Markers to Select Breast Cancer Patients for Dasatinib Therapy", MOLECULAR CANCER THERAPEUTICS, vol. 9, no. 5, 27 April 2010 (2010-04-27), pages 1120-1127, XP055096903, ISSN: 1535-7163, DOI: 10.1158/1535-7163.MCT-09-1117**
- **HUANG F ET AL: "Identification of candidate molecular markers predicting sensitivity in solid tumors to dasatinib: Rationale for patient selection", CANCER RESEARCH, AMERICAN ASSOCIATION FOR CANCER RESEARCH, US, vol. 67, no. 5, 1 March 2007 (2007-03-01), pages 2226-2238, XP002558115, ISSN: 0008-5472, DOI: 10.1158/0008-5472.CAN-06-3633**
- **WANG XI-DE ET AL: "Identification of candidate predictive and surrogate molecular markers for dasatinib in prostate cancer: rationale for patient selection and efficacy monitoring", GENOME BIOLOGY, BIOMED CENTRAL LTD., LONDON, GB, vol. 8, 29 November 2007 (2007-11-29), page R255, XP021041521, ISSN: 1465-6906**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 600 150 B1

Printed by Jouve, 75001 PARIS (FR)

**Description**

FIELD OF THE INVENTION

The present invention relates to a method for determining sensitivity of tumor cells to dasatinib. More particularly, the present invention relates to a method for determining sensitivity of tumor cells to dasatinib by evaluating the inhibitory capacity of a predetermined tyrosine kinase inhibitor in a measurement sample which is prepared from a biological sample containing the tumor cells. Further, the present invention relates to a computer program enabling a computer to execute the method.

BACKGROUND

Tyrosine kinase is a molecule which is involved in signaling for regulating cell differentiation and growth. Abnormalities of the tyrosine kinase are known to be causes of diseases such as cancer. The expression level of tyrosine kinase and its activity are excessively enhanced, particularly in tumor cells of various types of cancer, such as breast cancer and colon cancer. Accordingly, it is known that growth potential of tumor cells is activated by the excessive enhancement. Thus, a molecular target drug for targeting tyrosine kinase and inhibiting its activity has been studied and developed.

As one of the molecular target drugs, dasatinib is exemplified. Dasatinib is an anticancer drug that inhibits activities of various types of tyrosine kinases, such as BCR-ABL, Src, c-KIT, and PDGF receptors. Dasatinib is used as a therapeutic drug for chronic myelogenous leukemia (CML) which is resistant to imatinib (i.e., an effective therapeutic drug for CML), and relapsed or refractory Philadelphia chromosome positive acute lymphatic leukemia. Further, clinical application of dasatinib in breast cancer is also expected.

However, dasatinib is not always effective for all patients. This is described in the reference of Huang F. et al. (Cancer Res. 2007; 67 (5): 2226-2238). Huang F. et al. have suggested that, in various types of breast cancer-derived cell lines, some cell lines are sensitive to dasatinib and some cell lines are resistant to dasatinib. Therefore, a test to predict the sensitivity of patients under consideration for treatment with dasatinib to dasatinib allows unnecessary therapy to be avoided. Consequently, this is of great significance. Such prediction for the sensitivity is useful from the viewpoint of medical economy.

Currently, as the method for determining sensitivity to dasatinib, a method comprising: culturing breast cancer cells in the presence of dasatinib, examining a growth inhibition effect on the cells, and evaluating the effectiveness of dasatinib (disclosed by Huang F. et al.) is exemplified. However, it takes a long time to obtain results because the method is accompanied by a step of culturing cells. In order to take out tumor cells from biological samples such as tissues taken from patients and culture them equally, a skillful technique is required. Further, variations in results are easily caused depending on measurement environment. Accordingly, it is difficult to perform accurate tests.

As another method of determining sensitivity to dasatinib, there is a method described in the reference (Moulder S. et al., Mol Cancer Ther. 2010; 9 (5): 1120-1127). In the method, the gene expression levels of 19 kinds ofkinases which bind to dasatinib by a high affinity are measured with a microarray and then the sensitivity to dasatinib is predicted based on an average expression level. However, the microarray is expensive and it takes a long time for measurement. Therefore, it is difficult to promote the method as a test.

Document WO 2008/089135 discloses a correlation between sensitivity to one or more EGFR tyrosine kinase inhibitor and sensitivity to dasatinib.

SUMMARY OF THE INVENTION

The scope of the present invention is defined solely by the appended claims, and is not affected to any degree by the statements within this summary.

In view of the above circumstances, an object of the present invention is to provide a method for determining sensitivity of tumor cells to dasatinib whose operation is easy and which can be performed in a short time at low cost.

Surprisingly, the present inventors have found that there is a correlation between the sensitivity of tumor cells to dasatinib and the inhibitory capacity of a predetermined tyrosine kinase inhibitor in the measurement sample prepared from these tumor cells and completed the present invention.

That is, according to the present invention, there is provided a method for determining sensitivity of tumor cells to dasatinib which includes steps of:

preparing a measurement sample from a biological sample containing tumor cells;
evaluating the inhibitory capacity of at least one tyrosine kinase inhibitor selected from 4-(4'-phenoxylanilino)-6,7-dimethoxyquinazoline and 1-(1,1-dimethylethyl)-1-(4-methylphenyl)-1H-pyrazolo-[3,4-d]-pyrimidine-4-amine in the

measurement sample; and
determining sensitivity of tumor cells to dasatinib based on the evaluation results of the inhibitory capacity of the tyrosine kinase inhibitor.

According to the present invention, there is provided a computer program adapted to allow a computer to execute steps of:

acquiring the activity value of tyrosine kinase in a measurement sample prepared from a biological sample containing tumor cells in the presence of at least one tyrosine kinase inhibitor selected from 4-(4"-phenoxylanilino)-6,7-dimethoxyquinazoline and 1-(1,1-dimethylethyl)-1-(4-methylphenyl)-1H-pyrazolo-[3,4-d]-pyrimidine-4-amine;
evaluating the inhibitory capacity of the tyrosine kinase inhibitor based on the acquired activity value;
determining sensitivity of the tumor cells to dasatinib based on the evaluation result; and
outputting determination results.

According to the determination method, the sensitivity of tumor cells to dasatinib can be determined highly accurately and simply by measuring the tyrosine kinase activity in a measurement sample obtained from a biological sample containing tumor cells and evaluating levels of the inhibitory capacity of a predetermined tyrosine kinase inhibitor in the sample.

According to the computer program, it is possible to allow a computer to execute the determination method.

Based on the determination results obtained by the determination method, a doctor in clinical practice can obtain a more accurate index to determine whether dasatinib should be administered to the cancer patient in whom the biological sample is collected.

## BRIEF DESCRIPTION OF THE DRAWINGS

Fig. 1 is a block diagram showing a hardware configuration of a system for determining sensitivity to dasatinib;
Fig. 2 is a flow chart showing a process of determining sensitivity of tumor cells to dasatinib by a CPU 110a;
Fig. 3 shows graphs showing a distribution of relative activity values of various types of tyrosine kinase inhibitors in a dasatinib-sensitive cell line group and a dasatinib-insensitive cell line group; and
Fig. 4 shows a graph showing a distribution of the Dasatinib Target index in a dasatinib-sensitive cell line group and a dasatinib-insensitive cell line group.

## DETAILED DESCRIPTION OF THE PREFERRED EMBODIMENTS

The preferred embodiments of the present invention will be described hereinafter with reference to the drawings.

As described above, dasatinib (CAS number: 302962-49-8) is a compound which is known to inhibit activities of various types of tyrosine kinases in the art and is clinically used as an anticancer drug.

In the present specification, the term "sensitivity of tumor cells to dasatinib" means characteristics of tumor cells in which the survival and/or growth of tumor cells is suppressed or inhibited by effects of dasatinib. That is, the survival and/or growth of tumor cells with sensitivity to dasatinib is substantially suppressed or inhibited by dasatinib. On the other hand, the survival and/or growth of tumor cells without such sensitivity is not substantially suppressed or inhibited by dasatinib. In this regard, mechanisms in which the survival and/or growth of tumor cells is suppressed or inhibited by the effects of dasatinib include induction of cell death, such as apoptosis and necrosis.

In the method for determining sensitivity of tumor cells to dasatinib of the present invention (hereinafter simply referred to as "determination method"), a measurement sample is first prepared from a biological sample containing tumor cells.

The biological sample containing tumor cells may be a sample derived from a biological specimen or a sample derived from a culture obtained by culturing established tumor cells. Examples of the biological specimen include tissues such as tumor tissues, organ tissues, lymph node tissues, and blood, obtained from biological body (e.g., patients under consideration for cancer treatment with dasatinib) and celomic lavage fluid.

The tumor cells are not particularly limited as long as they are tumor cells which are known to be involved in abnormalities of tyrosine kinase, such as breast cancer, lung cancer, gastric cancer, colon cancer, kidney cancer, ovarian cancer, prostatic cancer, oral cancer, liver cancer, skin cancer, brain cancer, and leukemia cells. The breast cancer cells are preferred.

In the embodiments of the present invention, a measurement sample is prepared by obtaining a cellular fraction containing tyrosine kinase from a biological sample containing tumor cells. Preferably, a cellular fraction containing a

membrane-bound tyrosine kinase is obtained. Here, the membrane-bound tyrosine kinase is not particularly limited as long as it is a tyrosine kinase which is known in the art to be present in a cell in a state of being bound to a cell membrane. Hence, Src is preferred. Src is a type of non-receptor tyrosine kinase which is unevenly distributed in the body. Src binds to a cell membrane via its acyl chain and thus Src is present in a cell.

The cellular fraction containing tyrosine kinase can be prepared from the biological sample containing tumor cells by any known method in the art. Such a method is not particularly limited as long as it is a method for obtaining a cellular fraction containing an intracellular tyrosine kinase without impairing the enzyme activity. Preferably, the preparation is performed by a method for separating cytoplasm from the biological sample containing tumor cells, namely, a method for separating the cell membrane fraction to which tyrosine kinase is bound from cytoplasm. More preferably, the preparation is performed by a method including homogenizing the biological sample containing tumor cells in an appropriate buffer (hereinafter referred to as "homogenizing reagent"), obtaining an insoluble fraction from the thus obtained homogenate, mixing the insoluble fraction with a solubilizing solution containing a surfactant, and obtaining a soluble fraction from the obtained mixture.

The homogenate obtained from the homogenization using the homogenizing reagent can be separated into a soluble fraction (e.g., supernatant) and an insoluble fraction (e.g., pellet) by an appropriate method such as centrifugation. The soluble fraction contains cytoplasm-derived proteins and the like and the insoluble fraction contains cell membrane fragments which have various types of tyrosine kinases including receptor-type tyrosine kinases such as EGFR and HER2 and membrane-bound tyrosine kinases such as Src.

The mixture can be separated into a soluble fraction (e.g., supernatant) and an insoluble fraction (e.g., pellet) by an appropriate method such as centrifugation. The soluble fraction contains cell membranes having various tyrosine kinases which are solubilized with the surfactant (in micelles), and the insoluble fraction contains insoluble proteins, DNA and the like.

The homogenization of cells can be performed by a method for fragmenting a cell membrane. Examples of the method include known methods in the art, such as aspiration and discharge with a pipette, cell homogenization by freezing and thawing, mixing by a vortex mixer, homogenization with a blender, pressurization with a pestle, and sonication with a sonicator.

The homogenizing reagent may be used for preventing denaturation of tyrosine kinase during the homogenization of cells. The pH of the homogenizing reagent is not particularly limited so long as it is within a range in which tyrosine kinase can be recovered in a stable state without being denatured or deactivated. The pH of the homogenizing reagent is preferably from pH 4.0 to 9.0, more preferably from pH 4.5 to 8.5, still more preferably from pH 5.0 to 8.0.

Preferably, the homogenizing reagent contains a buffer. Examples of the buffer include a phosphoric acid buffer, an acetic acid buffer, a citric acid buffer, MOPS (3-morpholinopropanesulfonic acid), HEPES(2-[4-(2-hydroxyethyl)-1-piperazinyl]ethane sulfonic acid), TRIS(tris(hydroxymethyl)aminomethane), and TRICINE(N-[tris(hydroxymethyl)methyl] glycine).

The surfactant contained in the solubilized liquid is not particularly limited as long as it can solubilize the fragmented cell membrane into micelles and does not degrade and denature tyrosine kinase contained in the cell membrane. Since a surfactant with a charge may be bound to tyrosine kinase and change the conformation, it is preferable to use a nonionic surfactant which is not substantially bound to tyrosine kinase. Examples of the nonionic surfactant include surfactants having dodecyl ether, cetyl ether, stearyl ether, or p-t-octyl phenyl ether as a basic structure. Specific examples thereof include Nonidet P-40 (registered trademark of NP-40, Shell International Petroleum Company Limited), Triton-XR (registered trademark of Union Carbide Chemical and Plastics Inc.), Tween (registered trademark of ICI Americas Inc.), Brij (registered trademark of ICI Americas Inc.) and Emulgen (registered trademark of Kao Corporation.). The concentration of the surfactant in the solubilized liquid is usually from 0.05 to 5%, preferably from 0.1 to 3%, more preferably from 0.1 to 1%.

Preferably, the solubilized liquid contains the same buffer to be used for the homogenizing reagent. Further, the solubilized liquid preferably has a pH similar to that of the homogenizing reagent.

The homogenizing reagent and the solubilized liquid may contain a protease inhibitor, a phosphatase inhibitor, a reagent for preventing the oxidization of a mercapto group (SH group) (hereinafter referred to as "SH group stabilizer") or the like.

The protease inhibitor can be used in order to prevent the degradation of tyrosine kinases by proteases contained in cells. Examples of the protease inhibitor include metalloprotease inhibitors such as ethylenediaminetetraacetic acid (EDTA) and glycoletherdiaminetetraacetic acid (EGTA); serine protease inhibitors such as phenylmethylsulfonyl fluoride (PMSF), a trypsin inhibitor, and chymotrypsin; and cysteine protease inhibitors such as iodoacetamide and E-64. These protease inhibitors may be used alone or by mixing them.

A commercial product such as Protease Inhibitor Cocktail (Sigma) in which a plurality of protease inhibitors are premixed may be used.

The phosphatase inhibitor can be used in order to prevent a decrease in tyrosine kinase activities due to phosphatases contained in cells. Examples of the phosphatase inhibitor include sodium orthovanadate ($Na_3VO_4$), sodium

fluoride (NaF), and okadaic acid. These phosphatase inhibitors may be used alone or mixed with multiple inhibitors for use.

The SH group stabilizer can be used in order to prevent deactivation of tyrosine kinases. SH groups of enzymes are likely to be oxidized to form stable disulfides. The formation of disulfides may result in deactivation of enzymes due to the change in the enzyme conformation. Examples of the SH group stabilizer for preventing such oxidation of SH groups include reagents containing an SH group. Specific examples thereof include dithiothreitol (DTT), 2-mercaptoethanol, glutathione, cysteine, homocysteine, coenzyme A, and dihydrolipoic acid.

The concentration of the SH group stabilizer in the homogenizing reagent and/or solubilizing solution is preferably from 0.05 to 2 mM, more preferably from 0.07 to 1.7 mM, still more preferably from 0.1 to 1.5 mM in the case of DTT, for example. In the case of 2-mercaptoethanol, it is preferably from 0.1 to 15 mM, more preferably from 0.3 to 13 mM, still more preferably 0.5 to 12 mM.

In the preferable embodiment of the present invention, a cellular fraction containing tyrosine kinase prepared as a measurement sample includes a receptor-type tyrosine kinase with kinase activity and a membrane-bound tyrosine kinase.

For example, as for the cellular fraction containing tyrosine kinase, the receptor-type tyrosine kinase can form a polymer like homo- and hetero-dimers and can include tyrosine kinase held by the cell membrane in a state where the conformation is maintained at a degree that Src can form a complex with the polymer and/or in a state where Src is anchored to the cell membrane by an acyl chain added to a domain to be lipid-activated which is contained in Src.

The determination method of the present invention evaluates the inhibitory capacity of at least one tyrosine kinase inhibitor selected from 4-(4'-phenoxylanilino)-6,7-dimethoxyquinazoline (hereinafter also referred to as "Src kinase inhibitor I") and 1-(1,1-dimethylethyl)-1-(4-methylphenyl)-1H-pyrazolo-[3,4-d]-pyrimidine-4-amine (hereinafter also referred to as "PP 1 ") in the measurement sample. In the present specification, 4-(4'-phenoxylanilino)-6,7-dimethoxyquinazoline and 1-(1,1-dimethylethyl)-1-(4-methylphenyl)-1H-pyrazolo-[3,4-d]-pyrimidine-4-amine include their salts and solvates.

In the present specification, the term "inhibitory capacity of the tyrosine kinase inhibitor" means a degree that the enzyme activity of tyrosine kinase contained in the measurement sample is inhibited by the tyrosine kinase inhibitor. The Src kinase inhibitor I and PP1 are known as an ATP competitive inhibitor for Src, and they bind to the ATP bonding site of Src and inhibit the enzyme activity of Src. These Src inhibitors are marketed and commonly-available. The manufacturer and CAS number of the tyrosine kinase inhibitors are shown in Table 1.

[Table 1]

| Name | CAS number | Manufacturer |
|---|---|---|
| Src kinase inhibitor 1 | 179248-59-0 | Calbiochem |
| PP1 | 172889-26-8 | Biomol |

In the embodiments of the present invention, the Src kinase inhibitor I and PP1 may be used alone or by mixing them. Preferably, they are used alone.

Specifically, the inhibitory capacity of the tyrosine kinase inhibitor in the measurement sample is evaluated based on the activity value which is obtained by measuring the activity value of tyrosine kinase in the measurement sample in the presence of the tyrosine kinase inhibitor. Preferably, the inhibitory capacity of the tyrosine kinase inhibitor is evaluated based on the activity values which are obtained by measuring the activity value of tyrosine kinase in the measurement sample in the presence or absence of the inhibitor. In this case, the inhibitory capacity of the tyrosine kinase inhibitor is preferably evaluated based on a difference or a ratio of a tyrosine kinase activity value measured in the presence of the tyrosine kinase inhibitor (hereinafter also referred to as "first activity value") and a tyrosine kinase activity value measured in the absence of the tyrosine kinase inhibitor (hereinafter also referred to as "second activity value") in the measurement sample.

Examples of the value of the difference between the first and second activity values include a value obtained by subtracting the second activity value from the first activity value and a value obtained by subtracting the first activity value from the second activity value.

Examples of the ratio of the first and second activity values include a value obtained by dividing the first activity value by the second activity value and a value obtained by dividing the second activity value by the first activity value. The value obtained by dividing the first activity value by the second activity value is also referred to as "relative activity value".

The ratio of the first and the second activity values may be the inhibition rate calculated by using the relative activity value, i.e., a value (%) of an equation: "100-{(the first activity value)/(the second activity value)}x100".

The method for measuring the tyrosine kinase activity value is not particularly limited as long as it is a known method. The method for measuring the activity value of tyrosine kinase includes, for example, a method for detecting

autophosphorylation of tyrosine kinase. Specifically, cells are incubated, tyrosine kinase is recovered from cell membranes of the cultured cells, and autophosphorylation is detected using a phosphorylated tyrosine recognizing antibody which is labeled with a radioactive substance. Alternatively, the activity value of tyrosine kinase may be measured using a commercially available kit for performing the above method.

The first activity value is measured, for example, in the following manner. First, the measurement sample prepared in the above manner is brought into contact with the tyrosine kinase inhibitor. The contact is usually performed in a measurement sample solution. Then, the measurement sample brought into contact with the inhibitor is brought into contact with a substrate of tyrosine kinase and the phosphorylated substrate is detected to measure the first activity value.

The final concentration of the tyrosine kinase inhibitor during contact with the measurement sample may be suitably set according to the type of the inhibitor, the concentration of the substrate of tyrosine kinase, and the concentration of the phosphate group donor to be described later (e.g., ATP). For example, it may be set to a range of about 0.1 to 10 times based on the final concentration of ATP as the phosphate group donor.

The second activity value can be measured in the same manner as the first activity value except that the measurement sample is not brought into contact with the tyrosine kinase inhibitor.

The measurement of the first and second activity values is preferably performed by mixing the measurement sample, the substrate, and the phosphate group donor and detecting the substrate. The phosphate group of the phosphate group donor is incorporated into the substrate by a tyrosine kinase-mediated reaction so that the activity of tyrosine kinase can be measured by detecting the phosphorylated substrate.

The substrate for tyrosine kinase is preferably a substrate having low specificity to the type of tyrosine kinase (hereinafter referred to as "universal substrate") or a substrate having high specificity to a certain tyrosine kinase.

The substrate having high specificity to a certain tyrosine kinase, for example to EGFR, includes Grb2, myelin basic protein (MBP), histone H2B (HH2B), phospholipase C gamma and the like. The substrate having high specificity to EGFR may be a commercially available substrate such as GST-EGFR substrate (Stratagene). The GST-EGFR substrate is a fusion protein of glutathione-S-transferase (GST) and a substrate synthesized so as to be phosphorylated by the enzyme activity of EGFR.

Examples of the substrate having high specificity to Src include CDCP 1, p130Cas, paxillin, and cortactin. The substrate having high specificity to Src may be a substrate included in a commercially available kit such as ProFluor(trademark) Src-Family Kinase Assay (Promega KK.).

The above universal substrate is the substrate which has low specificity to the type of tyrosine kinase, i.e., the substrate for multiple types of tyrosine kinases. Thus, the universal substrate is preferably a known synthetic peptide which is synthesized so as to have low specificity to the type of tyrosine kinases. Specifically, such a synthetic peptide is preferably a peptide consisting of an amino acid sequence containing glutamic and tyrosine residues. The synthetic peptide may be, for example, a commercially available synthetic peptide such as Poly-Glu4-Tyr (biotin conjugate; UPSTATE). Other examples of the synthetic peptide include the synthetic peptides described as substrates for tyrosine kinase in such references as Norio Sasaki et al., The Journal of Biological Chemistry, Vol. 260, pages 9793-9804 (1985), Sergei Braun et al., The Journal of Biological Chemistry, Vol. 259, pages 2051-2054 (1984), and M. Abdel-Ghany et al., Proceeding of The National Academy of Science, Vol. 87, pages 7061-7065 (1990). The synthetic peptides disclosed in these references are consisting of amino acid sequences including a glutamic acid residue (Glu) and a tyrosine residue (Tyr) and synthesized so that Tyr is phosphorylated by two or more tyrosine kinases.

Specific examples of the amino acid sequences of the synthetic peptides include the followings:

an amino acid sequence in which a sequence consisting of four Glu's and one Tyr is repeated two times or more (hereinafter referred to as "amino acid sequence A");

an amino acid sequence in which a sequence consisting of one Glu and one Tyr is repeated two times or more (hereinafter referred to as "amino acid sequence B");

an amino acid sequence in which a sequence consisting of six Glu's, one Tyr and three alanine residues (Ala) is repeated two times or more (hereinafter referred to as "amino acid sequence C");

an amino acid sequence in which a sequence consisting of one Glu, one Tyr, and one Ala is repeated two times or more (hereinafter referred to as "amino acid sequence D"); and

an amino acid sequence in which a sequence consisting of two Glu's, one Tyr, six Ala's, and five lysine residues (Lys) is repeated two times or more (hereinafter referred to as "amino acid sequence E").

The reference (Tony Hunter, The Journal of Biological Chemistry, Vol. 257, pages 4843-4848 (1982)) reports that an acidic amino acid residue is important for the phosphorylation of Tyr by tyrosine kinase. Thus, the substrate is preferably the amino acid sequences A and C which contain more Glu's, acidic amino acid residues.

Examples of the phosphate group donor include adenosine triphosphate (ATP), adenosine 5'-O-(3-thiotriphosphate) (ATP-γS), 32 P-labeled adenosine 5'-O-(3-triphosphate) (γ-[32 P]-ATP), adenosine diphosphate (ADP), and adenosine monophosphate (AMP).

The measurement of the phosphorylated substrate is preferably carried out by separating the phosphorylated substrate from other proteins and detecting the substrate.

In order to separate the phosphorylated substrate from other proteins, the substrate may have an affinity tag. The use of the substrate having the affinity tag and a solid phase having a substance capable of binding to the affinity tag (hereinafter referred to as "binding substance") makes it possible to easily separate the phosphorylated substrate from other proteins and collect it. Specifically, the phosphorylated substrate can be collected by collecting a complex of the substrate and the solid phase and dissociating the link between the affinity tag and the binding substance of the solid phase in the complex.

The affinity tag is not specifically limited so long as it has a corresponding binding substance and it does not prevent the binding between the substrate and tyrosine kinase or phosphorylation of the substrate. As the affinity tag, for example, a polypeptide and hapten may be used. Specifically, GST, histidine, maltose binding protein, FLAG peptide (Sigma), Myc tag, hemagglutinin (HA) tag, Strep tag (IBA GmbH), biotin, avidin, streptavidin and the like may be used.

The substrate having the affinity tag may be the one obtained by chemically linking the affinity tag and the substrate. Alternatively, when the affinity tag is a polypeptide, the one obtained by introducing a vector having a recombinant gene encoding a fusion protein of the affinity tag and the substrate into a host and collecting the fusion protein produced by the host may also be used.

The binding substance corresponding to the affinity tag is not specifically limited so long as it can reversibly bind to the affinity tag. Examples of the binding substance include glutathione, nickel, amylose, anti-FLAG antibody (Sigma), anti-Myc antibody, anti-HA antibody, and Strep-Tactin (IBA GmbH).

The solid phase is not specifically limited so long as it is a support that can bind to the binging substance. Examples of the material for the solid phase include polysaccharides, plastics, and glass. Examples of the shape of the solid phase include beads and gel. Specific examples of the solid phase include Sepharose beads, agarose beads, magnetic beads, glass beads, and silicone gel. These sold phases may be charged in columns for use.

The combinations of the affinity tag and the solid phase having the binding substance include the following examples.

When the affinity tag is GST, the solid phase used may be glutathione Sepharose beads (hereinafter referred to as "glutathione beads"). The measurement of tyrosine kinase activity using this combination can be specifically carried out as follows. The cell membrane fraction having contacted with the tyrosine kinase inhibitor is brought into contact with the substrate linked to GST. By adding glutathione beads thereto, glutathione beads to which the phosphorylated substrate has been bound are obtained. After collecting these glutathione beads, reduced glutathione is added to dissociate the link between GST and glutathione beads and the substrate is collected.

Alternatively, the substrate linked to GST is first brought into contact with glutathione beads to obtain complexes of the substrate and the beads. Then, the cell membrane fraction having contacted with the tyrosine kinase inhibitor is brought into contact with the complexes and is collected. Reduced glutathione is added thereto to dissociate the link between GST and glutathione beads and the substrate is collected.

When the affinity tag is histidine, the solid phase having the binding substance can be nickel agarose beads, for example. The link between histidine and nickel can be dissociated with acid such as glycine-HCl or imidazole, for example.

When the affinity tag is maltose binding protein, the solid phase having the binding substance can be amylose magnetic beads, for example. The link between maltose binding protein and amylose can be dissociated with free amylose, for example.

When the affinity tag is FLAG peptide, the solid phase having the binding substance can be FLAG affinity gel (Sigma), for example. The link between FLAG peptide and FLAG affinity gel can be dissociated with acid such as glycine-HCl or 3×FLAG peptide (Sigma), for example.

When the affinity tag is Myc tag, the solid phase having the binding substance can be agarose beads bound to anti-Myc antibody.

When the affinity tag is HA tag, the solid phase having the binding substance can be agarose beads bound to anti-HA antibody.

The links between Myc tag and anti-Myc antibody and between HA tag and anti-HA antibody can be dissociated by denaturing proteins by adding acid or alkaline, for example. In these cases, the acid or alkaline to be used is preferably the one which allows the recovery of non-denatured protein. Specifically, the acid includes hydrochloric acid and alkaline includes sodium hydroxide.

When the affinity tag is Strep tag, the solid phase having the binding substance can be Strep-Tactin immobilized gel column (IBA GmbH), for example. The link between Strep tag and Strep-Tactin can be dissociated with desthiobiotin which reversibly react with streptavidin, for example.

After the contact of tyrosine kinase with the substrate, the enzyme reaction may be terminated by heating, cooling or addition of an enzyme inhibitor such as EDTA before collecting the phosphorylated substrate. By terminating

the enzyme reaction, variations in measurement results from sample to sample can be prevented due to the progression of the enzyme reaction during collection of the substrate.

It is preferable to measure the phosphorylated substrate by attaching a labeling substance to the phosphorylated substrate separated from the solid phase and detecting the labeling substance. The labeling substance includes, but not limited to, for example, fluorescent substances, enzymes, and radioisotopes. Examples of the fluorescent substances include fluorescein, coumarin, eosin, phenanthroline, pyrene, rhodamine, Cy3, Cy5, FITC, and Alexa Fluor (registered trademark). Examples of the enzymes include alkaline phosphatase and peroxidase. Examples of the radioisotopes include $^{32}P$, $^{33}P$, $^{131}I$, $^{125}I$, $^{3}H$, $^{14}C$, and $^{35}S$.

When the labeling substance is the enzyme, the detection may be carried out by detecting the coloring derived from the reaction of the enzyme with its substrate. When the enzyme is an alkaline phosphatase, the substrate includes a mixture of nitrotetrazolium blue chloride (NBT) and 5-bromo-4-chloro-3-indoxyl phosphate (BCIP). When the enzyme is peroxidase, the substrate includes diaminobenzidine (DAB).

The linkage between the phosphorylated substrate and the labeling substance may be performed in a known manner in the art. For example, the phosphorylated substrate can be linked to the labeling substance by using an antibody which has the labeling substance and can specifically bind to the phosphorylated substrate (hereinafter referred to as "phosphorylated substrate recognizing antibody").

The linkage between the phosphorylated substrate and the labeling substance may also be performed by using the phosphorylated substrate recognizing antibody and an antibody which is capable of binding to the phosphorylated substrate recognizing antibody and has the labeling substance (hereinafter referred to as "secondary antibody"). In this case, the labeling substance can substantially be linked to the phosphorylated substrate via the phosphorylated substrate recognizing antibody and the secondary antibody.

The linkage between the phosphorylated substrate and the labeling substance may also be performed by using the phosphorylated substrate recognizing antibody, the secondary antibody having biotin, and avidin having the labeling substance. In this case, the labeling substance can substantially be linked to the phosphorylated substrate via the phosphorylated substrate recognizing antibody, the secondary antibody, biotin, and avidin. The secondary antibody may have avidin and biotin may have the labeling substance.

The linkage between the phosphorylated substrate and the labeling substance may also be performed by using the phosphorylated substrate recognizing antibody having biotin and avidin having the labeling substance, or using the phosphorylated substrate recognizing antibody having avidin and biotin having the labeling substance.

When the labeling substance as above is used, the phosphorylated substrate can be detected by detecting the signal generated from the labeling substance, thereby allowing tyrosine kinase activity to be measured.

The phosphorylated substrate recognizing antibody and secondary antibody may be antibodies obtained by a conventionally-known method. Examples of such a method include a method for obtaining antibodies from blood of an animal immunized with an antigen and a method for obtaining antibodies by gene recombination. The antibodies may be polyclonal or monoclonal and may be a fragment of antibodies or a derivative thereof. A mixture of two or more of the antibodies may be used. Examples of the fragment and derivative of antibodies include Fab fragment, F(ab') fragment, F(ab) 2 fragment, and sFv fragment (Blazar et al., Journal of Immunology, 159: pages 5821-5833 (1997) and Bird et al., Science, 242: pages 423-426 (1988)). The antibody may belong to any class such as IgG and IgM.

The detection method of the phosphorylated substrate may be appropriately selected according to the type of the labeling substance. For example, when the labeling substance is the fluorescent substance or enzyme, the phosphorylated substrate can be detected by Western blotting. The phosphorylated substrate can be detected by blotting the phosphorylated substrate separated by electrophoresis such as SDS-PAGE to a membrane, incubating the membrane in a buffer containing the phosphorylated substrate recognizing antibody to allow for the binding between the antibody and the phosphorylated substrate, allowing the secondary antibody having the labeling substance to bind to the phosphorylated substrate recognizing antibody, and detecting the labeling substance. When the phosphorylated substrate has the affinity tag, the phosphorylated substrate can be measured in a similar manner to the case of using Western blotting by separating the phosphorylated substrate using the affinity tag and carrying out slot blotting instead of Western blotting.

When the labeling substance is the fluorescent substance, the phosphorylated substrate can also be detected by placing a solution containing the phosphorylated substrate in a tube, adding the phosphorylated substrate recognizing antibody having the fluorescent substance in order to allow for the binding of the antibody with the phosphorylated substance, and measuring the fluorescent intensity.

When the labeling substance is the enzyme, the phosphorylated substrate can be detected by enzyme linked immunosorbent assay (ELISA). ELISA includes direct adsorption and sandwich methods.

The direct adsorption method is a method including the direct adsorption of the phosphorylated substrate to the solid phase. The method may include, for example, adsorbing the phosphorylated substrate on the surface of the solid phase, allowing for the binding between the phosphorylated substrate recognizing antibody having the enzyme and the phosphorylated substrate, developing color by using the substrate to the enzyme of the phosphorylated substrate

recognizing antibody, and detecting the generated color.

The sandwich method is a method including detecting the phosphorylated substrate by using two phosphorylated substrate recognizing antibodies which recognize two different sites of the phosphorylated substrate. The method may include, for example, allowing the phosphorylated substrate recognizing antibody (hereinafter referred to as "solid phase antibody") to bind to the solid phase, allowing the phosphorylated substrate to bind to the sold phase antibody, allowing for the binding of the phosphorylated substrate recognizing antibody having the enzyme (hereinafter referred to as "labeling antibody") to the phosphorylated substrate, and detecting color generated by the reaction between the enzyme of the labeling antibody and the substrate to the enzyme.

When the labeling substance is the radioisotope, the phosphorylated substrate can be detected by radioimmunoassay (RIA). Specifically, the phosphorylated substrate recognizing antibody having the radioisotope is linked to the phosphorylated substrate and radiation is measured by a scintillation counter.

As described above, the first activity value is a kinase activity value measured in the presence of the tyrosine kinase inhibitor and the second activity value is a kinase activity value measured in the absence of the inhibitor, and thus the first activity value is usually predicted to be lower than the second activity value. However, the first activity value may be equal to or higher than the second activity value.

Therefore, in the case of using, for example, the value obtained by subtracting the first activity value from the second activity value as the value of the difference between the first and second activity values in the evaluation of the inhibitory capacity of the tyrosine kinase inhibitor, when the value of the difference is low (including zero and a negative value), the inhibitory capacity of the tyrosine kinase inhibitor can be evaluated to be low. On the other hand, when the value obtained by subtracting the first activity value from the second activity value is high, the inhibitory capacity can be evaluated to be high.

In the case of evaluating the inhibitory capacity of the tyrosine kinase inhibitor using, for example, the value obtained by dividing the first activity value by the second activity value as the ratio of the first and second activity value, when the ratio is high, the inhibitory capacity can be evaluated to be low. On the other hand, when the value obtained by dividing the first activity value by the second activity value is low, the inhibitory capacity can be evaluated to be high. Further, in the case of evaluating the inhibitory capacity of the tyrosine kinase inhibitor using the inhibition rate of the inhibitor in the measurement sample, i.e., the value of the equation: "100-{(the first activity value)/(the second activity value)}x100", when the value is low (including zero and a negative value), the inhibitory capacity can be evaluated to be low. On the other hand, when the inhibition rate is high, the inhibitory capacity can be evaluated to be high.

The evaluation of the inhibitory capacity of the tyrosine kinase inhibitor can be experientially performed by accumulating data of the first and second activity values regarding the sensitivity and insensitivity of tumor cells to the tyrosine kinase inhibitor. From the viewpoint of more accurate evaluation, it is preferable that the inhibitory capacity of the tyrosine kinase inhibitor in the measurement sample is evaluated based on the results obtained by comparing the difference or ratio of the first activity value and the second activity value with a threshold value to be described later.

That is, in the case of using, for example, the value obtained by subtracting the first activity value from the second activity value as the value of the difference between the first and second activity values in the above-described evaluation, when the value of the difference is compared with the threshold value and the value of the difference is lower than the threshold value, the inhibitory capacity of the tyrosine kinase inhibitor can be evaluated to be low. On the other hand, when the value obtained by subtracting the first activity value from the second activity value is compared with the threshold value and the value of the difference is more than the threshold value, the inhibitory capacity can be evaluated to be high.

In the case of using, for example, the value obtained by dividing the first activity value by the second activity value as the ratio of the first and second activity values, when the ratio is compared with the threshold value and the ratio is higher than the threshold value, the inhibitory capacity can be evaluated to be low. On the other hand, when the value obtained by dividing the first activity value by the second activity value is compared with the threshold value and the ratio is less or equal to the threshold value, the inhibitory capacity can be evaluated to be high. In the case of evaluating the inhibitory capacity of the inhibitor using the inhibition rate of the tyrosine kinase inhibitor in the measurement sample, i.e., the value of the equation: "100-{(the first activity value)/(the second activity value)}x100", when the inhibition rate is compared with the threshold value and the inhibition rate is lower than the threshold value, the inhibitory capacity can be evaluated to be low. On the other hand, when the inhibition rate is compared with the threshold value and the inhibition rate is more than the threshold value, the inhibitory capacity can be evaluated to be high.

The threshold value may be suitably set according to the type of the tyrosine kinase inhibitor and the type of tumor cells. The threshold value can be set, for example, based on the first and second activity values which are acquired by using tumor cells whose sensitivity to dasatinib is known and Src kinase inhibitor I or PP1 in the same manner as the above measurement of tyrosine kinase activity. When the threshold value is calculated as, for example, the inhibition rate of the inhibitor, the threshold value can be set in a range of usually 30 to 80%, preferably 35 to 75%, more preferably 40 to 70%.

In the determination method of the present invention, the sensitivity of tumor cells to dasatinib is determined

based on the evaluation result of the inhibitory capacity of the tyrosine kinase inhibitor.

In the preferred embodiment of the present invention, tumor cells are determined to have sensitivity to dasatinib when the inhibitory capacity of the tyrosine kinase inhibitor is evaluated as high and/or tumor cells are determined to have no sensitivity to dasatinib when the inhibitory capacity of the tyrosine kinase inhibitor is evaluated as low.

Alternatively, the sensitivity of tumor cells to dasatinib may be determined to be high when the inhibitory capacity of the tyrosine kinase inhibitor is evaluated as high and/or the sensitivity of tumor cells to dasatinib may be determined to be low when the inhibitory capacity of the tyrosine kinase inhibitor is evaluated as low. In the present specification, the levels of sensitivity of the tumor cells to dasatinib in the determination results may have the same meaning as the presence or absence of the sensitivity to dasatinib.

The computer program of the present invention will be described below.

The computer program of the present embodiment is a computer program adapted for allowing a computer to execute a method for determining sensitivity of tumor cells to dasatinib. Fig. 1 is a block diagram showing a hardware configuration of a system for determining sensitivity to dasatinib which includes a determination device 100 which executes the program of the present embodiment and a measurement device 200 for kinase activity in a measurement sample.

The system for determining sensitivity includes the determination device 100 and the measurement device 200 for kinase activity value which are connected by a cable 300. Data of the value of kinase activity which is measured by the measurement device 200 is sent to the determination device 100 via the cable 300. The determination device 100 is a device for analyzing the data output from the measurement device 200, determining sensitivity of tumor cells to dasatinib, and outputting determination results. The determination device 100 and the measurement device 200 are configured as an integrated device.

The determination device 100 is mainly configured by a main body 110, a display unit 120, and an input device 130. In the main body 110, the CPU (Central Processing Unit) 110a, a ROM (Read Only Memory) 110b, a RAM (Random Access Memory) 110c, a hard disk 110d, a read-out device 110e, and an input/output interface 110f, and an image output interface 110g are data-communicably connected by a bus 110h.

The CPU 110a can execute computer programs stored in the ROM 1101b and the computer programs loaded in the RAM 110c. The ROM 110b is configured by mask ROM, PROM, EPROM, EEPROM, and the like, and is recorded with computer programs to be executed by the CPU 110a, data used for the same.

The RAM 110c is configured by SRAM, DRAM, and the like. The RAM 110c is used to read out the computer programs recorded on the ROM 110b and the hard disc 110d. The RAM 110c is used as a work region when the CPU 110a executes these computer programs.

The hard disc 110d is installed with various computer programs to be executed by the CPU 110a such as operating system and application system program, as well as data used in executing the computer program. In order to allow the determination device 100 to realize the determination method of the present embodiment to be described later, the computer program 140a and a threshold value to be used for evaluation of the inhibitory capacity of the tyrosine kinase inhibitor are installed on the hard disk 110d.

The read-out device 110e is configured by a flexible disk drive, a CD-ROM drive or a DVD-ROM drive. The read-out device 110e is capable of reading out computer programs or data recorded on a portable recording medium 140. An application program 140a for allowing the computer to execute an operation is stored in the portable recording medium 140. It is also possible that the CPU 110a reads out the application program 140a from the portable recording medium 140 and installs the application program 140a on the hard disk 110d.

Operating system providing graphical interface environment such as Windows (registered trademark) manufactured and sold by US Microsoft Co. is installed in the hard disc 110d.

In the following explanation, the computer program 140a according to the determination of sensibility of dasatinib is configured to operate on the operating system.

The input/output interface 110f includes a serial interface such as USB, IEEE1394, and RS-232C; a parallel interface such as SCSI, IDE, and IEEE1284; and an analog interface such as D/A converter and A/D converter. The input/output interface 110f is connected to the input device 130 including a keyboard and a mouse. Thus, a user can use the input device 130 to input data of tyrosine kinase activity values (first and second activity values) obtained by measuring the measurement sample to the computer main body 110. The measurement device 200 which is capable of measuring tyrosine kinase activity in the measurement sample can be connected to the input/output interface 110f. In this case, the measurement device 200 can input the data of the first and second activity values to the computer main body 110.

The image output interface 110h is connected to the display unit 120 configured by LCD, CRT, or the like, and is configured to output an image signal corresponding to the image data provided from the CPU 110a to the display unit 120. The display unit 120 outputs image data according to the input image signal. The display unit 120 outputs image data provided from the CPU 110a to be described later.

Fig. 2 is a flow chart showing a process of determining sensitivity of tumor cells to dasatinib by the CPU 110a.

The first and second activity values are measured by bringing the measurement sample prepared from the biological sample containing tumor cells into contact with the substrate in the presence or absence of at least one tyrosine kinase inhibitor selected from the Src kinase inhibitor I and PP1 and detecting a signal generated from the phosphorylated substrate in the measurement sample brought into contact with the substrate by the measurement device 200.

The CPU 110a of the determination device 100 acquires the first and second activity values from the measurement device 200 via the input/output interface 110f (step S11) and allows the RAM 110c to store the acquired first and second activity values.

The CPU 110a reads out the first and second activity values stored in the RAM 110c, calculates a value of inhibitory capacity of the tyrosine kinase inhibitor (step S12), and allows the RAM110c to store the value.

The value of inhibitory capacity of the tyrosine kinase inhibitor is not particularly limited as long as it can evaluate the inhibitory capacity of the Src kinase inhibitor I or PP 1 in the measurement sample. Examples thereof include the value of the difference between the first and second activity values, the ratio of the first and second activity values, and the inhibition rate of the tyrosine kinase inhibitor. Examples of the difference of the first and second activity values include a value obtained by subtracting the first activity value from the second activity value and a value obtained by subtracting the second activity value from the first activity value. Examples of the ratio of the first and second activity values include the value obtained by dividing the first activity value by the second activity value and the value obtained by dividing the second activity value by the first activity value. The inhibition rate of the tyrosine kinase inhibitor includes the value (%) of the equation: "100-{(the first activity value)/(the second activity value)}x100".

In the present embodiment, the value (%) of the equation: "100-{(the first activity value)/(the second activity value)}x100" was calculated as the value of inhibitory capacity of the tyrosine kinase inhibitor.

The CPU 110a reads out a threshold value pre-stored in the hard disk 110d and compares the threshold value with the value of inhibitory capacity of the tyrosine kinase inhibitor (step S13). Here, the threshold value may be set depending on the value of inhibitory capacity of the tyrosine kinase inhibitor and it is not particularly limited. In the present embodiment, the threshold value of the value (%) of the equation: "100-{(the first activity value)/(the second activity value)}x100" was set to 44.9% for the Src kinase inhibitor I and 68.7% for PP1.

Here, the threshold value has been pre-stored in the hard disk 110d, however the present invention is not limited thereto. For example, the CPU 110a can receive data of the threshold value input from the input device via the input/output interface 110f. The CPU 110a can also receive data of the threshold value stored in an external storage device via the input/output interface 110f connected to the Internet. Further, the CPU 110a can also receive data of the threshold value recorded on the portable recording medium 140 by reading out it by the read-out device.

The CPU 110a determines that the tumor cells have sensitivity to dasatinib when the calculated value of inhibitory capacity of the tyrosine kinase inhibitor is more than the threshold value (step S14). The CPU 110a determines that the tumor cells have no sensitivity to dasatinib when the value of inhibitory capacity of the tyrosine kinase inhibitor is lower than the threshold value (step S 15). Here, examples of the value of inhibitory capacity of the tyrosine kinase inhibitor include the value obtained by subtracting the first activity value from the second activity value, the value obtained by dividing the second activity value by the first activity value, and the value (%) of the equation: "100-{(the first activity value)/(the second activity value)}x100".

The determination in steps S 14 and S15 may be suitably changed according to the value of inhibitory capacity of the tyrosine kinase inhibitor and it should not be limited to the above-described description. For example, when the value of inhibitory capacity of the tyrosine kinase inhibitor is the value obtained by subtracting the second activity value from the first activity value, the value obtained by dividing the first activity value by the second activity value or the value of the equation: "100-{(the first activity value)/(the second activity value)}x100", the CPU 110a can determine that the tumor cells have sensitivity to dasatinib when the value of inhibitory capacity of the tyrosine kinase inhibitor is lower than the threshold value. The CPU 110a can determine that the tumor cells have no sensitivity to dasatinib when the value of inhibitory capacity of the tyrosine kinase inhibitor is more than the threshold value.

The CPU 110a stores the determination results in the hard disk 110d and outputs them on the display unit 120 via the image output interface 110g (step S16).

In this case, the CPU 110a outputs only the determination result, however it may output instruction as to whether to provide treatment with dasatinib to a cancer patient in whom the biological sample containing tumor cells is collected. That is, the CPU 110a outputs the determination result and instruction not to provide treatment with dasatinib to the display unit 120 when determining that the tumor cells have no sensitivity to dasatinib. On the other hand, when determining that the tumor cells have sensitivity to dasatinib, the CPU 110a may output the determination result and instruction for providing treatment with dasatinib to the display unit 120.

EXAMPLE

[Example]

(1) Cell culture

Breast cancer cell lines used in this example are shown in Table 2. Penicillin (100 units/ml), Streptomycin (100 μg/ml), and Amphotericin B (0.25 μg/ml) are contained in all basal media shown in culture conditions. All of these cell lines can be purchased from American Type Culture Collection (ATCC). Further, basal medium, fetal bovine serum (FBS), bovine insulin, and glutathione which are used to culture the cell lines are marketed and commonly-available.

[Table 2]

| Cell line | ATCC Number | Basal media | FBS concentration (%) | Additives | Culture conditions |
|---|---|---|---|---|---|
| AU565 | CRL-2351 | RPMI 1640 | 10 | | 37°C, 5% $CO_2$ |
| BT-20 | HTB-19 | MEM | 10 | | 37°C, 5% $CO_2$ |
| BT-474 | HTB-20 | DMEM | 10 | | 37°C, 5% $CO_2$ |
| BT-549 | HTB-122 | RPMI 1640 | 10 | 1 μg/ml bovine insulin | 37°C, 5% $CO_2$ |
| HCC1419 | CRL-2326 | RPMI 1640 | 10 | | 37°C, 5% $CO_2$ |
| HCC1428 | CRL-2327 | RPMI 1640 | 10 | | 37°C, 5% $CO_2$ |
| HCC1806 | CRL-2335 | RPMI 1640 | 10 | | 37°C, 5% $CO_2$ |
| HCC1954 | CRL-2338 | RPMI 1640 | 10 | | 37°C, 5% $CO_2$ |
| HS578T | HTB-126 | DMEM | 10 | 10 μg/ml bovine insulin | 37°C, 5% $CO_2$ |
| MCF7 | HTB-22 | MEM | 10 | 10 μg/ml bovine insulin | 37°C, 5% $CO_2$ |
| MDA-MB-157 | HTB-24 | Leibovitz's L-15 | 10 | | 37°C 100% air |
| MDA-MB-231 | HTB-26 | Leibovitz's L-15 | 10 | | 37°C 100% air |
| MDA-MB-435S | HTB-129 | Leibovitz's L-15 | 10 | 10 μg/ml bovine insulin | 37°C 100% air |
| MDA-MB-436 | HTB-130 | Leibovitz's L-15 | 10 | 10 μg/ml bovine insulin, 10 μg/ml glutathione | 37°C 100% air |
| MDA-MB-453 | HTB-131 | Leibovitz's L-15 | 10 | | 37°C 100% air |
| MDA-MB-468 | HTB-132 | Leibovitz's L-15 | 10 | | 37°C 100% air |
| SK-BR-3 | HTB-30 | McCoy's 5a | 10 | | 37°C, 5% $CO_2$ |

(continued)

| Cell line | ATCC Number | Basal media | FBS concentration (%) | Additives | Culture conditions |
|---|---|---|---|---|---|
| ZR-75-1 | CRL-1500 | RPMI 1640 | 10 | | 37°C, 5% $CO_2$ |
| ZR-75-30 | CRL-1504 | RPMI 1640 | 10 | | 37°C, 5% $CO_2$ |

Breast cancer cell lines were cultured under the conditions shown in Table 2 so as to be about 80% confluent in a 150-mm dish. Thereafter, the cells were recovered using PBS and a scraper, and a cell pellet was obtained by centrifugal operation (at 900xg, for 5 minutes, at 4°C). The cell pellet was frozen with liquid nitrogen and stored at -80°C before preparation of the measurement sample.

As for the above-described cell lines, their sensitivities to dasatinib have been examined by Huang F. et al. (Cancer Res. 2007; 67 (5): 2226-2238). The above-described cell lines were classified into two groups: dasatinib-sensitive and -insensitive cell lines based on IC50 values in the proliferation assay according to the description in the reference of Huang F. et al. The cell lines classified into each group are shown in Table 3 below.

[Table 3]

<table>
<tr><td>Sensitive cell line</td><td></td><td>Insensitive cell line</td></tr>
<tr><td>BT-20</td><td></td><td>AU565</td></tr>
<tr><td>HCC1806</td><td></td><td>BT-474</td></tr>
<tr><td>HCC1954</td><td></td><td>BT-549</td></tr>
<tr><td>HS578T</td><td></td><td>HCC1419</td></tr>
<tr><td>MDA-MB-157</td><td></td><td>HCC1428</td></tr>
<tr><td>MDA-MB-231</td><td></td><td>MCF7</td></tr>
<tr><td></td><td></td><td>MDA-MB-435S</td></tr>
<tr><td></td><td></td><td>MDA-MB-436</td></tr>
<tr><td></td><td></td><td>MDA-MB-453</td></tr>
<tr><td colspan="3">MDA-MB-468</td></tr>
<tr><td colspan="3">SK-BR-3</td></tr>
<tr><td colspan="3">ZR-75-1</td></tr>
<tr><td colspan="3">ZR-75-30</td></tr>
</table>

(2) Preparation of measurement sample

500 μl of NP40(-) solubilized buffer (20 mM HEPES (pH 7.4)), 10 (w/v) % glycerol, 0.2% protease inhibitor cocktail (#P8340: Sigma-Aldrich (product composition: 104 mM AEBSF, 80 μM aprotinin, 4 mM bestatin, 1.4 mM E-64, 2 mM leupeptin, 1.5 mM Pepstatin A), 50 mM NaF and 200 μM $Na_3VO_4$) was added to the cell pellet, followed by stirring for 2 seconds three times using a vortex mixer. After being allowed to stand for 10 minutes, the soluble fraction was removed by centrifugal operation (at 20000xg, for 30 minutes, at 4°C). The obtained pellet was resuspended in 500 μl of NP40(-) solubilized buffer, the soluble fraction was again removed by centrifugal operation (at 20000xg, for 20 minutes, at 4°C), and the pellet was washed. The pellet was resuspended by adding 500 μl of NP40(+) solubilized buffer (a buffer prepared by adding NP-40 to the NP40(-) solubilized buffer at a final concentration of 1% to the pellet, pipetting 50 times, and vortexing for 2 seconds three times. After being allowed to stand for 10 minutes, a cell membrane fraction containing a membrane protein was obtained by centrifugal operation (at 20000xg, for 40 minutes, at 4°C). The protein concentration of the obtained cell membrane fraction was measured by DC protein assay (#500-0116 JA: Bio-Rad). Then, the cell membrane fraction was used as a measurement sample for the following experiments. The measurement sample was frozen with liquid nitrogen and stored at -80°C before use for the following kinase activity measurement.

(3) Evaluation of inhibitory capacity of tyrosine kinase inhibitor in measurement sample

NeutrAvidin Coated Plates (HBC) (96-well white plate; #15509: Pierce) were washed three times with 300 μl of TBS-T (20 mM Tris-HCl (pH 7.4)), 150 mM NaCl, 0.1% tween). Then, 100 μl of Poly-Glu4-Tyr biotin conjugate (#12440: Upstate) which had been diluted to 1 μg/ml with TBS (20 mM Tris-HCl (pH 7.4), 150 mM NaCl) was added to the wells. After shaking at 37°C and 400 rpm for 90 minutes, the wells were washed with 300 μl of TBS-T three times and a measuring plate was obtained.

The measurement sample was diluted with a kinase buffer (20 mM HEPES (pH 7.4), 25 mM $MgCl_2$, 1.25 mM $MnCl_2$, 250 μM $Na_3VO_4$, 12.5 (w/v) % glycerol) so as to adjust the protein concentration to 6.25 μg/ml. The diluted measurement sample was transferred to wells of a 96 well V bottom plate. As the tyrosine kinase inhibitors, Src kinase inhibitor I known as an inhibitor of Src (CAS No. 179248-59-0: Calbiochem), PP1 (CAS No. 172889-26-8: Biomol), and Src kinase inhibitor II (Calbiochem) represented by Structural formula (I) below were used. A DMSO solution (inhibitor concentration: 5mM) containing these inhibitors (Inhibitors 1 to 10 shown in Table 6) was added to the wells at a ratio of 1:160 based on the diluted measurement sample, followed by mixing thereof. As the control, DMSO was added to the diluted measurement sample at the same ratio as the solution of the inhibitor.

Structural formula (I)

[Formula 1]

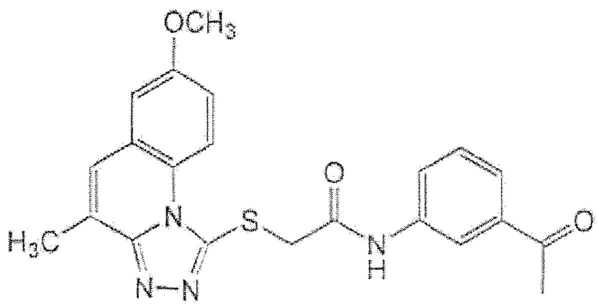

After addition of each of the tyrosine kinase inhibitors, a mixed solution of the measurement sample and the inhibitor was allowed to stand at room temperature for 10 minutes. 40 μl of the mixed solution was transferred to wells of the measuring plate and 10 μl of an ATP solution (20 mM HEPES (pH 7.4), 125 mM ATP) as the phosphate group donor was added to the wells. The composition of the solution in the wells is as follows: protein concentration: 5 μg/ml, 20 mM HEPES (pH 7.4), 20 mM $MgCl_2$, 1 mM $MnCl_2$, 200 μM $Na_3VO_4$, 10 (w/v) % glycerol, and 25 μM ATP, 25 μM kinase inhibitor.

After shaking the measuring plate at 37°C and 400 rpm for 60 minutes, the solution was removed from the measuring plate, and the wells were washed with 300 μl of TBS-T three times. 100 μl of Starting Block T20 (TBS) (#37543: Pierce) as a blocking solution was added to the wells, followed by blocking at 37°C and 400 rpm for 10 minutes. The blocking solution was removed from the measuring plate. 100 μl of a solution prepared by diluting HRP-labeled anti-phosphorylated tyrosine antibody (#sc-508 HRP: Santa Cruz) with Starting Block T20 (TBS) at a ratio of 1:1000 was added to the wells, followed by shaking at 37°C and 400 rpm for 90 minutes. Then, the wells were washed with 300 μl of TBS-T five times. 100 μl of SuperSignal ELISA Pico Chemiluminescent Substrate (#37070: Pierce) as a substrate was added to the wells, followed by shaking at room temperature for 1 minute. Thereafter, the emission from the wells was measured by a GENios microplate reader (manufactured by Tecan Trading AG). A ratio of the measurement value of tyrosine kinase activity in the presence of the inhibitors and the measurement value of the control (in the absence of the inhibitors) was calculated as a relative activity value (hereinafter also referred to as "RA"). Specifically, the RA was calculated based on the equation below. The RA of the dasatinib-sensitive cell line and the RA of the dasatinib-insensitive cell line are shown in Tables 4 and 5.

(RA) = (activity value measured in the presence of the inhibitors)/ (activity value measured in the absence of the inhibitors)

[Table 4]

| Dasatinib-sensitive cell line | RA | | |
|---|---|---|---|
| | Src kinase inhibitor I | PP1 | Src kinase inhibitor II |
| BT-20 | 0.434 | 0.309 | 0.668 |
| HCC1806 | 0.329 | 0.160 | 0.476 |
| HCC1954 | 0.535 | 0.292 | 0.644 |
| HS578T | 0.418 | 0.194 | 0.709 |
| MDA-MB-157 | 0.397 | 0.173 | 0.715 |
| MDA-MB-231 | 0.364 | 0.134 | 0.426 |

[Table 5]

| Dasatinib-insensitive cell line | RA | | |
|---|---|---|---|
| | Src kinase inhibitor I | PP1 | Src kinase inhibitor II |
| AU565 | 0.632 | 0.302 | 0.731 |
| BT-474 | 0.624 | 0.496 | 0.620 |
| BT-549 | 0.394 | 0.193 | 0.528 |
| HCC1419 | 0.637 | 0.446 | 0.615 |
| HCC1428 | 0.563 | 0.451 | 0.720 |
| MCF7 | 0.508 | 0.295 | 0.307 |
| MDA-MB-435S | 0.551 | 0.313 | 0.500 |
| MDA-MB-436 | 0.551 | 0.403 | 0.514 |
| MDA-MB-453 | 0.837 | 0.448 | 0.549 |
| MDA-MB-468 | 0.190 | 0.322 | 0.523 |
| SK-BR-3 | 0.567 | 0.260 | 0.414 |
| ZR-75-1 | 0.442 | 0.314 | 0.697 |
| ZR-75-30 | 0.627 | 0.507 | 0.596 |

Based on data shown in Tables 4 and 5 above, a boxplot showing a distribution of relative activity values of the tyrosine kinase inhibitors in groups of the dasatinib-sensitive and -insensitive cell lines was produced. The produced boxplot is shown in Fig. 3. In Fig. 3, p represents a significant probability in the Student's t-test.

As is apparent from Fig. 3, in the case of the Src kinase inhibitor I and PP1, the relative activity value in the dasatinib-sensitive cell line tends to be low, while the relative activity value in the dasatinib-insensitive cell line tends to be high. On the other hand, in the case of the Src kinase inhibitor II, a significant difference in distribution of relative activity values between dasatinib-sensitive and -insensitive cell lines was not observed.

Therefore, it was shown that the sensitivity of tumor cells to dasatinib could be predicted by evaluating the inhibitory capacity of the Src kinase inhibitor I or PP 1 based on the activity value of tyrosine kinase in the measurement sample.

(Comparative examples)

The determination method of the present invention was compared with the method described in the reference (Moulder S. et al., Mol Cancer Ther. 2010; 9 (5): A 1120-1127) and the determination accuracy of each of the methods was examined.

Moulder S. et al. have used the Dasatinib Target index (T index) which is defined based on the gene expression level measured with the microarray as a determination index for sensitivity to 19 kinds of kinases which bind with a high affinity to dasatinib.

The T index was reproduced based on microarray data (GSE6569) of cell lines described and published by Moulder S. et al. and a boxplot showing a distribution of the T index in groups of the dasatinib-sensitive and -insensitive cell lines was produced. The produced boxplot is shown in Fig. 4. In Fig. 4, p represents a significant probability in the Student's t-test.

From Fig. 4, it was confirmed that the T index in the dasatinib-sensitive cell line tended to be high, while the T index in the dasatinib-insensitive cell line tended to be low.

The determination performance of the determination method of the present invention and the method described by Moulder S. et al. was examined by ROC analysis. Areas under the curve (AUC) of ROC curves acquired by the analysis are shown in Table 6. The AUC is an index showing that the determination with high accuracy can be achieved as the value is close to 1. For each index, the threshold was determined by the maximal Youden Index defined as "sensitivity + specificity-1". The sensitivity, specificity, positive predictive value (PPV), and negative predictive value (NPV) were evaluated. These results are shown in Table 6.

[Table 6]

| | AUC | Sensitivity (%) | Specificity (%) | PPV (%) | NPV (%) |
|---|---|---|---|---|---|
| RA of Src kinase inhibitor I | 0.846 | 100 | 69.2 | 60 | 100 |
| RA of PP1 | 0.910 | 100 | 69.2 | 60 | 100 |
| Dasatinib Target index | 0.885 | 100 | 69.2 | 60 | 100 |

As is apparent from Table 6, the determination method of the present invention based on the inhibitory capacity of the tyrosine kinase inhibitor showed an AUC equal to a method based on the determination index derived from the gene expression level of kinase. That is, it was showed that the determination method of the present invention had determination performance almost equal to the method described by Moulder S. et al.

The foregoing detailed description and examples have been provided by way of explanation and illustration, and are not intended to limit the scope of the appended claims. Many variations in the presently preferred embodiments will be obvious to one of ordinary skill in the art, and remain within the scope of the appended claims and their equivalents.

## Claims

1. A method for determining sensitivity of tumor cells to dasatinib comprising steps of:

   preparing a measurement sample from a biological sample containing tumor cells;
   evaluating inhibitory capacity of at least one tyrosine kinase inhibitor selected from 4-(4'-phenoxylanilino)-6,7-dimethoxyquinazoline and 1-(1,1-dimethylethyl)-1-(4-methylphenyl)-1H-pyrazolo-[3,4-d]-pyrimidine-4-amine in the measurement sample; and
   determining sensitivity of tumor cells to dasatinib based on the evaluation results of the inhibitory capacity of the tyrosine kinase inhibitor.

2. The method according to claim 1, wherein the tumor cells are breast cancer cells.

3. The method according to claim 1 or 2, wherein the preparation process is a process of obtaining a cellular fraction containing a membrane-bound tyrosine kinase from a biological sample containing tumor cells to prepare a measurement sample.

4. The method according to claim 3, wherein the cellular fraction is a membrane fraction.

5. The method according to any one of claims 1 to 4, wherein the evaluation process is a process of measuring the activity value of tyrosine kinase in the measurement sample in the presence of the tyrosine kinase inhibitor and evaluating the inhibitory capacity of the tyrosine kinase inhibitor based on the acquired activity value.

6. The method according to claim 5, wherein the evaluation process is a process of further measuring the activity value of tyrosine kinase in the measurement sample in the absence of the tyrosine kinase inhibitor.

7. The method according to claim 6, wherein the evaluation process is a process of acquiring a difference or a ratio of the activity value measured in the presence of the tyrosine kinase inhibitor and the activity value measured in the absence of the tyrosine kinase inhibitor and evaluating the inhibitory capacity of the tyrosine kinase inhibitor based on the acquired value.

8. The method according to claim 7, wherein the evaluation process is a process of comparing the acquired value with a threshold and evaluating the inhibitory capacity of the tyrosine kinase inhibitor based on the compared result.

9. The method according to any one of claims 1 to 8, wherein the determination process is a process of determining that the tumor cells have sensitivity to dasatinib when the inhibitory capacity of the tyrosine kinase inhibitor is evaluated as high and/or determining that the tumor cells have no sensitivity to dasatinib when the inhibitory capacity of the tyrosine kinase inhibitor is evaluated as low.

10. A computer program product adapted for enabling a computer to determine sensitivity of tumor cells to dasatinib comprising:

a computer readable, medium and
software, instructions, on the computer readable medium, for enabling the computer to perform predetermined operations comprising:

acquiring the activity value of tyrosine kinase in a measurement sample prepared from a biological sample containing tumor cells in the presence of at least one tyrosine kinase inhibitor selected from 4-(4'-phenoxylanilino)-6,7-dimethoxyquinazoline and 1-(1,1-dimethylethyl)-1-(4-methylphenyl)-1H-pyrazolo-[3,4-d]-pyrimidine-4-amine;
evaluating the inhibitory capacity of the tyrosine kinase inhibitor based on the acquired activity value;
determining sensitivity of the tumor cells to dasatinib based on the evaluation result; and
outputting determination results.

11. The computer program product according to claim 10, wherein the acquiring operation further acquires the activity value of tyrosine kinase in the measurement sample in the absence of the tyrosine kinase inhibitor.

12. The computer program product according to claim 11, further comprising an operation to calculate the value showing the inhibitory capacity of the tyrosine kinase inhibitor based on the activity value acquired in the acquiring operation, wherein the evaluation operation is an operation which evaluates the inhibitory capacity of the tyrosine kinase inhibitor based on the value showing the calculated inhibitory capacity.

13. The computer program product according to claim 12, wherein the calculation operation is an operation which calculates a difference or a ratio of the activity value in the presence of the tyrosine kinase inhibitor and the activity value in the absence of the tyrosine kinase inhibitor as the value showing the inhibitory capacity of the tyrosine kinase inhibitor, and the evaluation operation is an operation which compares the value showing the inhibitory capacity with a threshold and evaluates the inhibitory capacity based on the compared result.

14. The computer program product according to any one of claims 10 to 13, wherein the determination operation is an operation which determines that the tumor cells have sensitivity to dasatinib when the inhibitory capacity of the tyrosine kinase inhibitor is evaluated as high and/or determines that the tumor cells have no sensitivity to dasatinib when the inhibitory capacity of the tyrosine kinase inhibitor is evaluated as low.

## Patentansprüche

1. Verfahren zur Bestimmung der Empfindlichkeit von Tumorzellen gegen Dasatinib, umfassend die Schritte:

Herstellen einer Messprobe aus einer biologischen Probe, die Tumorzellen enthält;
Evaluieren der inhibitorischen Fähigkeit mindestens eines Tyrosinkinaseinhibitors, ausgewählt aus 4-(4'-Phenoxylanilin)-6,7-dimethoxychinazolin und 1-(1,1-Dimethylethyl)-1-(4-methylphenyl)-1H-pyrazol-[3,4-d]-pyrimidin-4-amin, in der Messprobe; und
Bestimmen der Empfindlichkeit von Tumorzellen gegen Dasatinib auf Grundlage der Evaluierungsergebnisse der inhibitorischen Fähigkeit des Tyrosinkinaseinhibitors.

**2.** Verfahren gemäß Anspruch 1, wobei die Tumorzellen Brustkrebszellen sind.

**3.** Verfahren gemäß Anspruch 1 oder 2, wobei das Herstellungsverfahren ein Verfahren des Erhaltens einer zellulären Fraktion ist, die eine membrangebundene Tyrosinkinase aus einer biologischen Probe, die Tumorzellen enthält, enthält, um eine Messprobe herzustellen.

**4.** Verfahren gemäß Anspruch 3, wobei die zelluläre Fraktion eine Membranfraktion ist.

**5.** Verfahren gemäß mindestens einem der Ansprüche 1 bis 4, wobei das Evaluierungsverfahren ein Verfahren des Messens des Aktivitätswerts von Tyrosinkinase in der Messprobe in der Gegenwart des Tyrosinkinaseinhibitors und des Evaluierens der inhibitorischen Fähigkeit des Tyrosinkinaseinhibitors auf Grundlage des erfassten Aktivitätswerts ist.

**6.** Verfahren gemäß Anspruch 5, wobei das Evaluierungsverfahren ein Verfahren des zusätzlichen Messens des Aktivitätswerts der Tyrosinkinase in der Messprobe in der Abwesenheit des Tyrosinkinaseinhibitors ist.

**7.** Verfahren gemäß Anspruch 6, wobei das Evaluierungsverfahren ein Verfahren des Erfassens eines Unterschieds oder eines Verhältnisses des Aktivitätswerts, gemessen in der Gegenwart des Tyrosinkinaseinhibitors, und des Aktivitätswertes, gemessen in der Abwesenheit des Tyrosinkinaseinhibitors, und des Evaluierens der inhibitorischen Fähigkeit des Tyrosinkinaseinhibitors auf Grundlage des erfassten Werts ist.

**8.** Verfahren gemäß Anspruch 7, wobei das Evaluierungsverfahren ein Verfahren des Vergleichens des erfassten Werts mit einem Schwellenwert und des Evaluierens der inhibitorischen Fähigkeit des Tyrosinkinaseinhibitors auf Grundlage des verglichenen Ergebnisses ist.

**9.** Verfahren gemäß mindestens einem der Ansprüche 1 bis 8, wobei das Bestimmungsverfahren ein Verfahren des Bestimmens ist, dass die Tumorzellen Empfindlichkeit gegen Dasatinib haben, wenn die inhibitorische Fähigkeit des Tyrosinkinaseinhibitors als hoch evaluiert wird, und/oder des Bestimmens ist, dass die Tumorzellen keine Empfindlichkeit gegen Dasatinib haben, wenn die inhibitorische Fähigkeit des Tyrosinkinaseinhibitors als niedrig evaluiert wird.

**10.** Computerprogramm-Erzeugnis, angepasst dazu, einen Computer dazu zu befähigen, die Empfindlichkeit von Tumorzellen gegen Dasatinib zu bestimmen, umfassend:

ein vom Computer lesbares Medium und
Software, Anweisungen auf dem vom Computer lesbaren Medium, um den Computer zu befähigen, vorbestimmte Operationen durchzuführen, umfassend:

Erfassen des Aktivitätswerts von Tyrosinkinase in einer Messprobe, hergestellt aus einer biologischen Probe, die Tumorzellen enthält, in der Gegenwart mindestens eines Tyrosinkinaseinhibitors, ausgewählt aus 4-(4'-Phenoxylanilin)-6,7-dimethoxychinazolin und 1-(1,1-Dimethylethyl)-1-(4-methylphenyl)-1H-pyrazol-[3,4-d]-pyrimidin-4-amin;
Evaluieren der inhibitorischen Fähigkeit des Tyrosinkinaseinhibitors auf Grundlage des erfassten Aktivitätswerts;
Bestimmen der Empfindlichkeit der Tumorzellen gegen Dasatinib auf Grundlage des Evaluierungsergebnisses; und
Ausgeben der Ergebnisse der Bestimmung.

**11.** Computerprogramm-Erzeugnis gemäß Anspruch 10, wobei die Operation des Erfassens zusätzlich den Aktivitätswert der Tyrosinkinase in der Messprobe in der Abwesenheit des Tyrosinkinaseinhibitors erfasst.

**12.** Computerprogramm-Erzeugnis gemäß Anspruch 11, ferner umfassend eine Operation, um den Wert zu berechnen, der die inhibitorische Fähigkeit des Tyrosinkinaseinhibitors zeigt, auf Grundlage des Aktivitätswerts, der in der Operation des Erfassens erfasst wurde, wobei die Operation des Evaluierens eine Operation ist, welche die inhibitorische Fähigkeit des Tyrosinkinaseinhibitors auf Grundlage des Werts evaluiert, der die berechnete inhibitorische Fähigkeit zeigt.

**13.** Computerprogramm-Erzeugnis gemäß Anspruch 12, wobei die Operation des Berechnens eine Operation ist, welche einen Unterschied oder ein Verhältnis des Aktivitätswerts in der Gegenwart des Tyrosinkinaseinhibitors und des Aktivitätswerts in der Abwesenheit des Tyrosinkinaseinhibitors als den Wert berechnet, der die inhibitorische Fähigkeit des Tyrosinkinaseinhibitors zeigt, und die Operation des Evaluierens eine Operation ist, die den Wert, der die inhibitorische Fähigkeit zeigt, mit einem Schwellenwert vergleicht und die inhibitorische Fähigkeit auf Grundlage des verglichenen Ergebnisses evaluiert.

**14.** Computerprogramm-Erzeugnis gemäß mindestens einem der Ansprüche 10 bis 13, wobei die Operation des Bestimmens eine Operation ist, die bestimmt, dass die Tumorzellen Empfindlichkeit gegen Dasatinib haben, wenn die inhibitorische Fähigkeit des Tyrosinkinaseinhibitors als hoch evaluiert wird, und/oder bestimmt, dass die Tumorzellen keine Empfindlichkeit gegen Dasatinib haben, wenn die inhibitorische Fähigkeit des Tyrosinkinaseinhibitors als niedrig evaluiert wird.

## Revendications

**1.** Procédé de détermination de la sensibilité de cellules tumorales au dasatinib comprenant les étapes qui consistent :

à préparer un échantillon de mesure à partir d'un échantillon biologique contenant des cellules tumorales ;
à évaluer une capacité inhibitrice d'au moins un inhibiteur de la tyrosine kinase choisi parmi 4-(4'-phénoxylanilino)-6,7-diméthoxyquinazoline et 1-(1,1-diméthyléthyl)-1-(4-méthylphényl)-1H-pyrazolo[3,4-d]-pyrimidine-4-amine dans l'échantillon de mesure ; et
à déterminer la sensibilité de cellules tumorales au dasatinib sur la base des résultats d'évaluation de la capacité inhibitrice de l'inhibiteur de la tyrosine kinase.

**2.** Procédé selon la revendication 1, dans lequel les cellules tumorales sont des cellules de cancer du sein.

**3.** Procédé selon la revendication 1 ou 2, dans lequel le processus de préparation est un processus d'obtention d'une fraction cellulaire contenant une tyrosine kinase liée à la membrane à partir d'un échantillon biologique contenant des cellules tumorales pour préparer un échantillon de mesure.

**4.** Procédé selon la revendication 3, dans lequel la fraction cellulaire est une fraction membranaire.

**5.** Procédé selon l'une quelconque des revendications 1 à 4, dans lequel le processus d'évaluation est un processus de mesure de la valeur d'activité de la tyrosine kinase dans l'échantillon de mesure en présence de l'inhibiteur de la tyrosine kinase et d'évaluation de la capacité inhibitrice de l'inhibiteur de la tyrosine kinase sur la base de la valeur d'activité acquise.

**6.** Procédé selon la revendication 5, dans lequel le processus d'évaluation est un processus de mesure en outre de la valeur d'activité de la tyrosine kinase dans l'échantillon de mesure en l'absence de l'inhibiteur de la tyrosine kinase.

**7.** Procédé selon la revendication 6, dans lequel le processus d'évaluation est un processus d'acquisition d'une différence ou d'un rapport de la valeur d'activité mesurée en présence de l'inhibiteur de la tyrosine kinase et de la valeur d'activité mesurée en l'absence de l'inhibiteur de la tyrosine kinase et d'évaluation de la capacité inhibitrice de l'inhibiteur de la tyrosine kinase sur la base de la valeur acquise.

**8.** Procédé selon la revendication 7, dans lequel le processus d'évaluation est un processus de comparaison de la valeur acquise à un seuil et d'évaluation de la capacité inhibitrice de l'inhibiteur de la tyrosine kinase sur la base du résultat comparé.

**9.** Procédé selon l'une quelconque des revendications 1 à 8, dans lequel le processus de détermination est un processus de détermination que les cellules tumorales ont une sensibilité au dasatinib lorsque la capacité inhibitrice de l'inhibiteur de la tyrosine kinase est évaluée comme étant élevée et/ou de détermination que les cellules tumorales n'ont pas de sensibilité au dasatinib lorsque la capacité inhibitrice de l'inhibiteur de la tyrosine kinase est évaluée comme étant faible.

**10.** Produit de programme informatique adapté pour permettre à un ordinateur de déterminer la sensibilité de cellules tumorales au dasatinib, comprenant :

un support lisible par ordinateur, et
un logiciel, des instructions, sur le support lisible par ordinateur, pour permettre à l'ordinateur d'exécuter des opérations prédéterminées comprenant :

l'acquisition de la valeur d'activité de la tyrosine kinase dans un échantillon de mesure préparé à partir d'un échantillon biologique contenant des cellules tumorales en présence d'au moins un inhibiteur de la tyrosine kinase choisi parmi 4-(4'-phénoxylanilino)-6,7-diméthoxyquinazoline et 1-(1,1-diméthyléthyl)-1-(4-méthylphényl)-1H-pyrazolo-[3,4-d]-pyrimidine-4-amine ;
l'évaluation de la capacité inhibitrice de l'inhibiteur de la tyrosine kinase sur la base de la valeur d'activité acquise ;
la détermination de la sensibilité des cellules tumorales au dasatinib sur la base du résultat d'évaluation ; et
la sortie des résultats de détermination.

**11.** Produit de programme informatique selon la revendication 10, dans lequel l'opération d'acquisition acquiert en outre la valeur d'activité de la tyrosine kinase dans l'échantillon de mesure en l'absence de l'inhibiteur de la tyrosine kinase.

**12.** Produit de programme informatique selon la revendication 11, comprenant en outre une opération de calcul de la valeur représentant la capacité inhibitrice de l'inhibiteur de la tyrosine kinase sur la base de la valeur d'activité acquise dans l'opération d'acquisition, où l'opération d'évaluation est une opération qui évalue la capacité inhibitrice de l'inhibiteur de la tyrosine kinase sur la base de la valeur représentant la capacité inhibitrice calculée.

**13.** Produit de programme informatique selon la revendication 12, dans lequel l'opération de calcul est une opération qui calcule une différence ou un rapport de la valeur d'activité en présence de l'inhibiteur de la tyrosine kinase et de la valeur d'activité en l'absence de l'inhibiteur de la tyrosine kinase comme étant la valeur représentant la capacité inhibitrice de l'inhibiteur de la tyrosine kinase, et l'opération d'évaluation est une opération qui compare la valeur représentant la capacité inhibitrice à un seuil et qui évalue la capacité inhibitrice sur la base du résultat comparé.

**14.** Produit de programme informatique selon l'une quelconque des revendications 10 à 13, dans lequel l'opération de détermination est une opération qui détermine que les cellules tumorales ont une sensibilité au dasatinib lorsque la capacité inhibitrice de l'inhibiteur de la tyrosine kinase est évaluée comme étant élevée et/ou qui détermine que les cellules tumorales n'ont pas de sensibilité au dasatinib lorsque la capacité inhibitrice de l'inhibiteur de la tyrosine kinase est évaluée comme étant faible.

FIG. 1

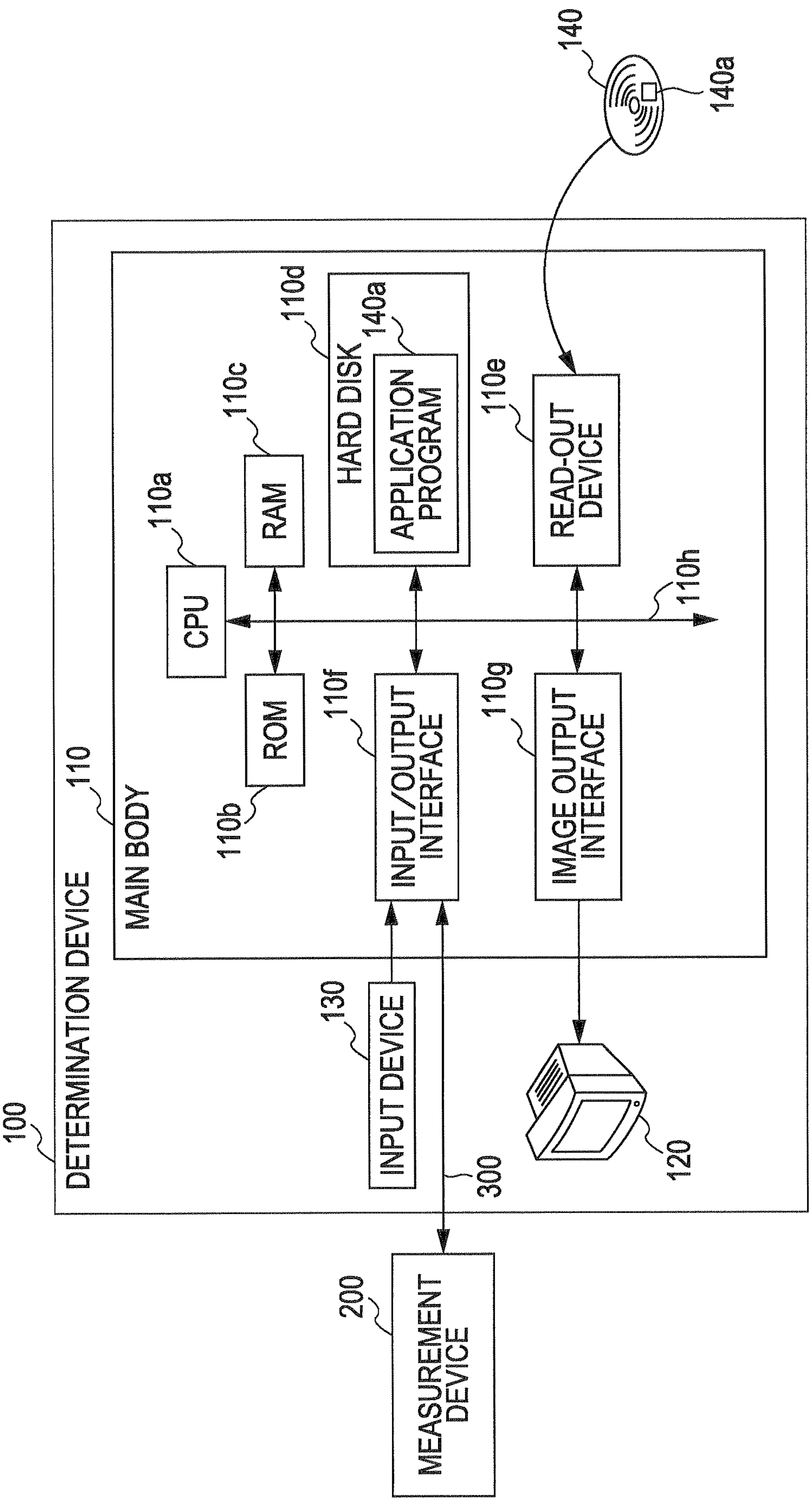
140
140a
100
DETERMINATION DEVICE
110
MAIN BODY
110b
ROM
110a
CPU
110c
RAM
110d
HARD DISK
140a
APPLICATION PROGRAM
110e
READ-OUT DEVICE
110h
110f
INPUT/OUTPUT INTERFACE
110g
IMAGE OUTPUT INTERFACE
130
INPUT DEVICE
300
120
200
MEASUREMENT DEVICE

FIG. 2

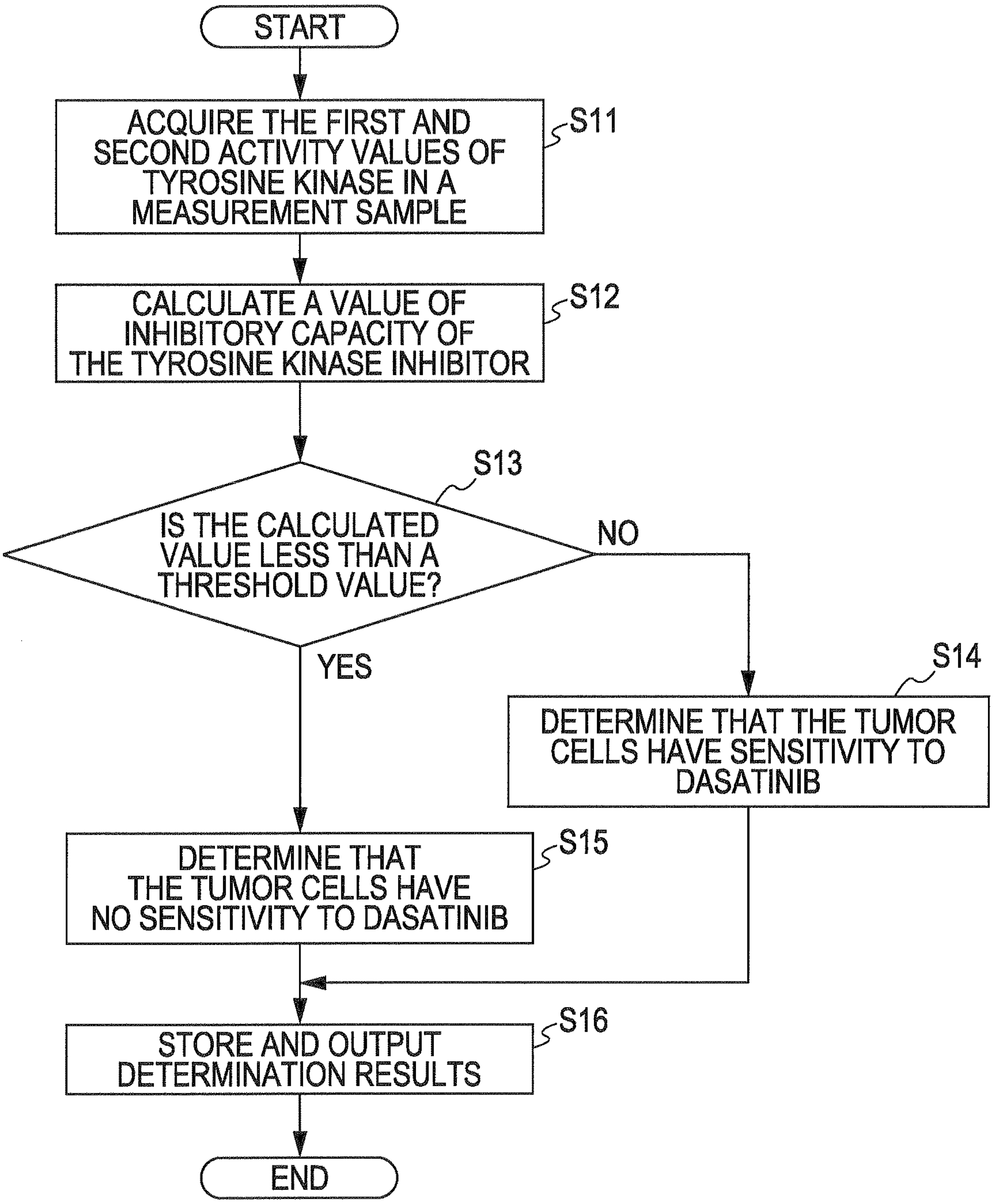
START
ACQUIRE THE FIRST AND SECOND ACTIVITY VALUES OF TYROSINE KINASE IN A MEASUREMENT SAMPLE
S11
CALCULATE A VALUE OF INHIBITORY CAPACITY OF THE TYROSINE KINASE INHIBITOR
S12
S13
IS THE CALCULATED VALUE LESS THAN A THRESHOLD VALUE?
NO
YES
S14
DETERMINE THAT THE TUMOR CELLS HAVE SENSITIVITY TO DASATINIB
DETERMINE THAT THE TUMOR CELLS HAVE NO SENSITIVITY TO DASATINIB
S15
STORE AND OUTPUT DETERMINATION RESULTS
S16
END

FIG. 3

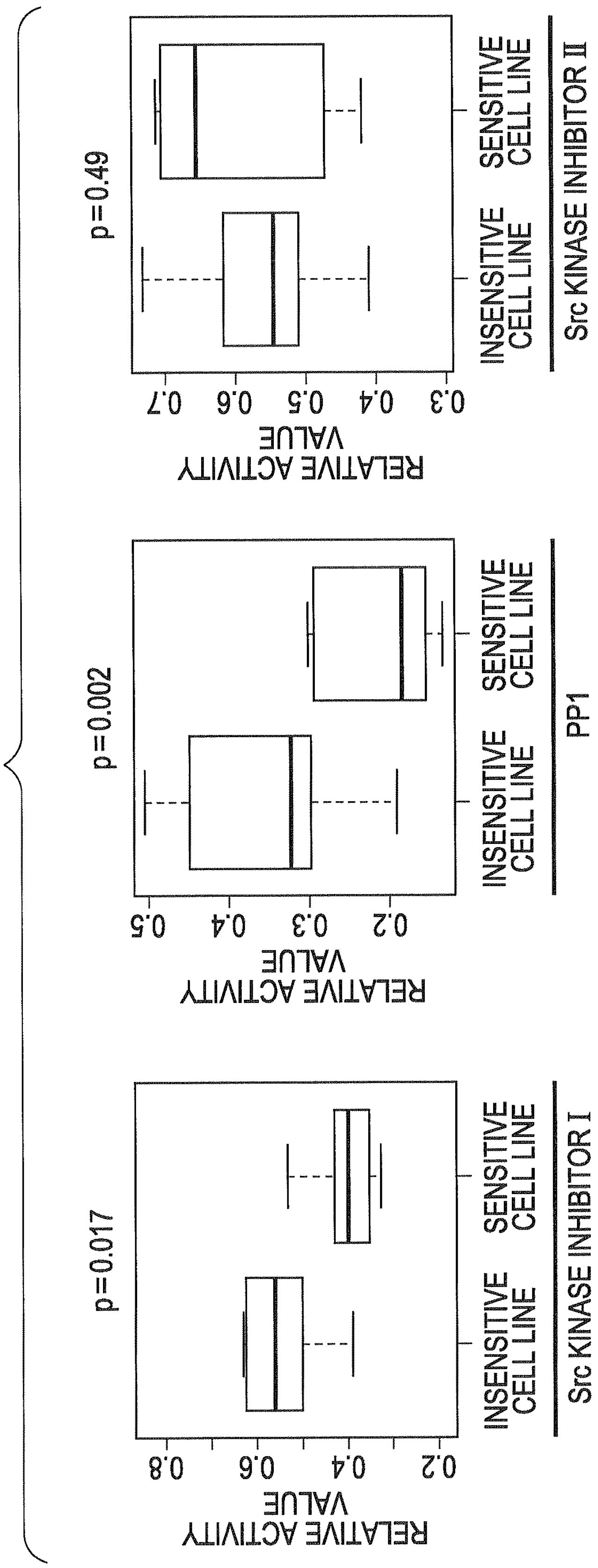
p=0.017
RELATIVE ACTIVITY VALUE
0.2
0.4
0.6
0.8
INSENSITIVE CELL LINE
SENSITIVE CELL LINE
Src KINASE INHIBITOR I
p=0.002
RELATIVE ACTIVITY VALUE
0.2
0.3
0.4
0.5
INSENSITIVE CELL LINE
SENSITIVE CELL LINE
PP1
p=0.49
RELATIVE ACTIVITY VALUE
0.3
0.4
0.5
0.6
0.7
INSENSITIVE CELL LINE
SENSITIVE CELL LINE
Src KINASE INHIBITOR II

FIG. 4

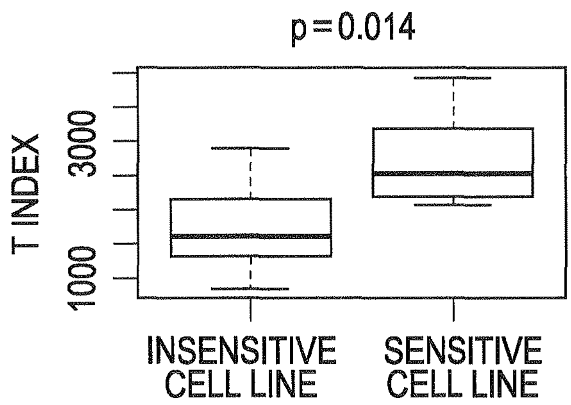
p=0.014
T INDEX
3000
1000
INSENSITIVE CELL LINE
SENSITIVE CELL LINE

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008089135 A


### Non-patent literature cited in the description


- **HUANG F. et al.** *Cancer Res.,* 2007, vol. 67 (5), 2226-2238
- **MOULDER S. et al.** *Mol Cancer Ther.,* 2010, vol. 9 (5), 1120-1127
- **SASAKI et al.** *The Journal of Biological Chemistry,* 1985, vol. 260, 9793-9804
- **SERGEI BRAUN et al.** *The Journal of Biological Chemistry,* 1984, vol. 259, 2051-2054
- **M. ABDEL-GHANY et al.** *Proceeding of The National Academy of Science,* 1990, vol. 87, 7061-7065
- **TONY HUNTER.** *The Journal of Biological Chemistry,* 1982, vol. 257, 4843-4848
- **BLAZAR et al.** *Journal of Immunology,* 1997, vol. 159, 5821-5833
- **BIRD et al.** *Science,* 1988, vol. 242, 423-426
- **HUANG F et al.** *Cancer Res.,* 2007, vol. 67 (5), 2226-2238
- **MOULDER S. et al.** *Mol Cancer Ther.,* 2010, vol. 9 (5), A 1120-1127